# DEMANDE DE BREVET EUROPEEN

(11) **EP 4 574 149 A1**
(43) Date de publication de la demande: **25.06.2025**
(21) Numéro de dépôt: 23307371.7
(22) Date de dépôt: 22.12.2023
(51) Int. Cl.: A61K 31/436, A61K 9/00, A61P 1/16, A61P 41/00

(54) **COMPOSITION DE RAPAMYCINE, DE DIMÉTHYLSULFOXIDE ET D'EAU POUR UTILISATION DANS LE TRAITEMENT DE LA STÉATOSE D'UN GREFFON HÉPATIQUE**

(71) Demandeur: Assistance Publique - Hôpitaux de Paris, 75012 Paris (FR); Institut National de la Santé et de la Recherche Médicale, 75013 Paris (FR); Sorbonne Université, 75006 Paris (FR); Université Paris Cité, 75006 Paris (FR)
(72) Inventeur: SCATTON, Olivier, 92260 FONTENAY-AUX-ROSES (FR); TURCO, Célia, 25000 BESANCON (FR); BOUDY, Vincent, 75013 PARIS (FR); ROSA, Fréderic, 77400 THORIGNY-SUR-MARNE (FR); TOUSSAINT, Balthazar, 94210 SAINT-MAUR-DES-FOSSES (FR)
(74) Mandataire: Santarelli

(57) **Abrégé**

La présente invention porte sur une composition comprenant de la rapamycine, du diméthylsulfoxide et de l'eau pour utilisation dans le traitement de la stéatose d'un greffon hépatique. L'invention a également pour objet une composition comprenant de la rapamycine, du diméthylsulfoxide et de l'eau susceptible d'être obtenue par un procédé de préparation ainsi qu'un procédé d'administration. L'invention concerne également l'utilisation d'une composition comprenant de la rapamycine, du diméthylsulfoxide et de l'eau pour traiter la stéatose d'un greffon hépatique.

## Description

### DOMAINE DE L'INVENTION

La présente invention se rapporte à une composition comprenant de la rapamycine, du diméthylsulfoxide et de l'eau pour utilisation dans le traitement de la stéatose d'un greffon hépatique. L'invention vise également un procédé de préparation et un procédé d'administration de ladite composition ainsi que son utilisation pour traiter la stéatose d'un greffon hépatique.

### ARRIERE-PLAN TECHNOLOGIQUE

La transplantation hépatique (TH) reste le seul traitement curatif pour la cirrhose au stade terminal comme pour certains cancers primitifs du foie à un stade peu évolué. Les principales indications sont le carcinome hépatocellulaire et la cirrhose décompensée. Depuis plusieurs années, le nombre de transplantations hépatiques n'a cessé d'augmenter avec en parallèle, des résultats à long terme qui sont en constante amélioration. Cependant, la pénurie d'organes reste une limitation importante à la transplantation hépatique et est à l'origine d'une mortalité significative sur liste d'attente, évaluée entre 10% et 20%. En 2 ans, les données rapportent l'absence de baisse de l'incidence des décès pour 1000 patients*année malgré une augmentation de 5,6% de greffes (Agence de la Biomédecine).

Devant cette pénurie d'organes, les équipes prélèvent de plus en plus de foies issus de donneurs âgés et/ou présentant des « critères élargis ».

En France, la stéatose est la première cause de refus des greffons hépatiques. En 2021, sur 336 foies proposés et non prélevés, près de 72 greffons ont été refusés pour leur aspect stéatosique (c'est-à-dire avec une accumulation de lipides dans les hépatocytes). De même, sur 34 foies prélevés et non greffés, la moitié d'entre eux ne l'ont pas été à cause de leur aspect stéatosique.

En pratique, les greffons présentant une stéatose de plus de 30% sont jugés non transplantables. En effet, ces greffons sont plus fragiles et présentent un risque augmenté de dysfonction en raison d'un seuil de tolérance à l'ischémie-reperfusion très bas.

Pourtant, l'utilisation de ce type de greffon stéatosique permettrait de greffer 10% de malades supplémentaires chaque année. De plus, devant l'augmentation constante de la prévalence de l'obésité (15% à 35% de la population occidentale), le nombre de greffons stéatosiques devrait continuer de croître ces prochaines années.

Des approches novatrices sont actuellement explorées pour augmenter le nombre et améliorer la qualité de ces greffons. Ces méthodes visent à diminuer l'écart entre le nombre de receveurs et le pool d'organes disponibles. Une de ces approches est la préservation dynamique (PM) qui est une procédure au cours de laquelle les organes sont perfusés ex *vivo* après leur prélèvement. Les objectifs de la PM sont multiples et comprennent l'atténuation des lésions d'ischémie-reperfusion (IR), la capacité de prolonger le temps de préservation ex *vivo,* l'amélioration de l'état et de la fonction des organes, ainsi que l'évaluation de la viabilité des organes avant la transplantation.

Actuellement, il n'existe aucune technique de préservation ou de traitement pharmacologique validés permettant de traiter la stéatose d'un greffon hépatique.

Il existe donc un besoin en traitements de la stéatose d'un greffon hépatique. En effet, toute solution thérapeutique qui permettrait d'utiliser des greffons hépatiques jugés initialement non transplantables serait une avancée majeure afin d'augmenter le pool d'organes disponibles et de diminuer ainsi la mortalité des patients sur liste d'attente.

### RESUME DE L'INVENTION

Un premier objet de la présente invention porte sur une composition comprenant de la rapamycine, du diméthylsulfoxide et de l'eau pour utilisation dans le traitement de la stéatose d'un greffon hépatique.

Il a particulièrement été mis en évidence que l'administration d'une telle composition ex *vivo* au greffon hépatique, par exemple en perfusion en condition normothermique, pourrait permettre de traiter une stéatose, supérieure à 30%, d'un greffon hépatique jugé initialement non transplantable.

L'invention a également pour objet ladite composition susceptible d'être obtenue par un procédé de préparation comprenant les étapes suivantes :
1) Mise en solution de rapamycine avec du diméthylsulfoxide à une température supérieure à 18°C;
2) Ajout d'eau à une température supérieure à 18°C, à la solution de rapamycine et de diméthylsulfoxide obtenue à l'étape 1;
3) Stérilisation de la solution obtenue à l'étape 2, de préférence par filtration.

L'invention concerne également un procédé d'administration de ladite composition, comportant les étapes suivantes :
1) Installation d'un greffon hépatique sur une machine de perfusion en condition hypothermique, puis en condition normothermique;
2) Injection dans le circuit de perfusion de la composition comprenant de la rapamycine, du diméthylsulfoxide et de l'eau.

L'invention concerne également l'utilisation d'une composition comprenant de la rapamycine, du diméthylsulfoxide et de l'eau pour traiter la stéatose d'un greffon hépatique.

### DESCRIPTION DETAILLEE

Un premier objet de la présente invention porte sur une composition comprenant de la rapamycine, du diméthylsulfoxide et de l'eau pour utilisation dans le traitement de la stéatose d'un greffon hépatique.

Dans le cadre de la présente invention, les termes « traiter », « traitement » etc. indiquent une amélioration du pourcentage de stéatose sur le greffon hépatique.

La stéatose, communément appelée « maladie du foie gras » correspond à l'accumulation de graisse dans le foie. La stéatose est directement liée au surpoids et à l'obésité.

De préférence, la stéatose du greffon hépatique est supérieure à 30%. Le degré de stéatose permet de classer les greffons en trois groupes :
- Moins de 30% : greffon transplantable,
- Entre 30% et 60% : greffon potentiellement transplantable, après traitement (perfusion, traitement pharmacologique) et évaluation de la viabilité;
- Plus de 60% : greffon non transplantable.

La rapamycine, également nommée sirolimus a été initialement découverte comme un métabolite antifongique produit par *Streptomyces hygroscopicus* à partir d'un échantillon de sol de l'île de Pâques. Les propriétés immunosuppressives et antiprolifératives de cette molécule vis-à-vis des cellules de mammifères ont suscité un intérêt pour l'identification de son mécanisme d'action (Li J et al., 2014).

Il s'agit d'un inhibiteur de mTOR (mechanistic target of rapamycin) qui cible la protéine kinase mTORC1 et inactive cette voie de signalisation.

La voie de signalisation mTOR est complexe et les conséquences de cette inhibition sur les fonctions cellulaires sont multiples, incluant deux grandes actions :
- Rôle dans le métabolisme hépatique lipidique via des propriétés « dégraissantes » dites « defatting », notamment la lipogenèse, l'homéostasie des triglycérides et l'autophagie. La rapamycine est utilisée en prévention du rejet d'organe et est associée à une augmentation du cholestérol (hypercholestérolémie) et des triglycérides sériques (hypertriglycéridémie) faisant l'objet d'une surveillance biologique.
- Action immunologique : la fonction et la production de cellules immunitaires. Cette voix est particulièrement importante en transplantation car l'inhibition du complexe mTOR/FKBP-12 bloque la prolifération des lymphocytes T et B en réponse à l'IL-2 et l'IL-15 ce qui permet de limiter le rejet d'allogreffe (utilisation en clinique dans la prophylaxie du rejet des greffes).

La rapamycine correspond au principe actif de la composition selon l'invention.

Le diméthylsufoxide ou DMSO est un composé organosulfuré et un solvant aprotique polaire. Ce composé est un agent de solubilisation.

De préférence, la composition selon l'invention est sous forme de solution injectable.

Par « solution », on entend une composition liquide dépourvue de particules visibles, en utilisant la procédure selon la Pharmacopée Européenne et/ou américaine.

Par « solution injectable », on entend une solution qui satisfait aux conditions des Pharmacopées Européenne et/ou américaine et qui est suffisamment liquide pour être injectée *in vivo* ou ex *vivo,* de préférence dans un circuit de perfusion.

La rapamycine est un produit instable en solution. Il n'existe à ce jour aucun produit pharmaceutiquement acceptable contenant de la rapamycine en solution. Cette forme est indispensable pour pouvoir être injectée dans un contexte de conditionnement hépatique en vue d'une greffe.

La composition selon l'invention a notamment pour but d'être utilisée dans le traitement de la stéatose d'un greffon hépatique au sein d'un bloc opératoire (à température ambiante). Cependant, le diméthylsulfoxide a un point de fusion de 18,5°C pouvant entrainer des problèmes d'utilisation notamment au bloc opératoire car le produit serait à l'état solide (la température ambiante étant proche du point de fusion). Par ailleurs, l'eau a un point de fusion de 0°C. Avantageusement, le mélange diméthylsulfoxide et eau permet un abaissement du point de congélation de la composition. En effet, le point de fusion de la composition est inférieur à -18,5°C, permettant ainsi d'obtenir une composition possédant une stabilité physicochimique améliorée comparativement à une composition comprenant seulement du DMSO. Ainsi, la composition selon la présente invention est stable lors du stockage et de l'utilisation.

Cette composition permet la stabilisation de la rapamycine sous forme de solution prête à l'emploi. Ainsi, l'injection de la composition de rapamycine sous forme de solution peut être réalisée au moment jugé le plus opportun par les équipes de greffes spécialisées.

Dans un premier mode de réalisation, la composition selon l'invention est utilisée dans une administration ex vivo au greffon hépatique, notamment lors d'une perfusion sur machine.

Plusieurs types de protocoles de conservation des greffons hépatiques avant transplantation et de perfusion ex *vivo* ont été proposés, à savoir la perfusion hypothermique oxygénée ou HOPE (4°C), la conservation en normothermie continue ou cNMP (37°C) et la perfusion sub-normothermique (21°C) (Muller X et al., 2022). En pratique, le mode de perfusion le plus efficace sur le plan thérapeutique, donnant les meilleurs résultats, consiste à associer plusieurs conditions en débutant par une perfusion en condition hypothermique suivie d'un réchauffement progressif jusqu'en normothermie (Boteon YL et al., décembre 2018). La première phase en hypothermie oxygénée permet la reconstitution des stocks d'Adenosine TriphosPhate (ATP) avec un apport d'oxygène en conditions de métabolisme réduit tandis que la deuxième en normothermie à 37°C permet le maintien de l'organe en conditions physiologiques et donc a l'avantage de pouvoir tester la viabilité de l'organe et de réaliser des interventions pharmacologiques.

De préférence, l'administration ex *vivo* est une perfusion en condition normothermique.

On entend par « condition normothermique », une température comprise entre 36°C et 38°C.

De préférence, l'administration de la composition selon l'invention s'effectue en bolus dans un circuit de perfusion ex *vivo* avec la circulation continue d'un milieu adapté (culots globulaires, albumine, milieu tampon nutritif pour cellules du type solution de Krebs) en condition normothermique pendant 12 heures à une température comprise entre 36°C et 38°C.

Il est à noter que l'administration dans un circuit de perfusion ex *vivo* permet de s'affranchir du risque de toxicité systémique, la rapamycine étant délivrée uniquement dans l'organe. Il permet également d'augmenter les doses sans risque de toxicité extra-hépatique. La normothermie garantit un fonctionnement physiologique et métabolique normal du foie et permet ainsi de réaliser des contrôles sur la qualité du foie et des études pharmacologiques spécifiques de la rapamycine (pharmacocinétique et pharmacodynamie).

Par ailleurs, la composition selon l'invention est efficace sur les greffons hépatiques sans altération de la viabilité cellulaire.

De préférence, la concentration en rapamycine dans la composition est inférieure ou égale à 1 mg/mL, de préférence entre 0,4 mg/mL et 1 mg/mL, encore plus préférentiellement de 0,5 mg/mL.

De préférence, la concentration circulante en rapamycine est comprise entre 120 ng/mL et 360 ng/mL, de préférence de 180 ng/mL.

De préférence, la composition selon l'invention comprend entre 0,02% et 0,5% de rapamycine, exprimé en pourcentage massique.

De préférence, ladite composition comprend de l'eau ultra-pure, également nommée « eau » dans la présente invention.

L'eau ultra-pure est une eau qui a été purifiée en respectant des niveaux élevés de spécification. En règle générale, l'eau ne contient que du H₂0, ainsi qu'un nombre équilibré d'ions H⁺ et OH⁻. Elle a une résistivité de 18,2 MO.cm, un COT (Carbone Organique Total) < 10 ppb et un nombre de bactéries <10 UFC/ml. Pour être classée comme ultra-pure, l'eau ne doit contenir aucune endotoxine détectable.

De préférence, la composition selon l'invention comprend entre 70% et 99% de diméthylsulfoxide et entre 1% et 30% d'eau, de préférence 85% de diméthylsulfoxide et 15% d'eau, exprimé en pourcentage volumique.

Encore plus préférentiellement, la composition selon l'invention comprend 0,5 mg/mL de rapamycine, 85% de diméthylsulfoxide et 15% d'eau, exprimé en pourcentage volumique.

De préférence, le diméthylsulfoxide et l'eau sont pharmaceutiquement acceptables.

Avantageusement, le ratio diméthylsulfoxide/eau est compris entre 2,5 et 95, de préférence entre 2,6 et 92 et encore plus préférentiellement entre 2,65 et 91,25.

De préférence, la composition selon l'invention est conservée entre -30°C et - 10°C, de préférence à -20°C.

La composition selon l'invention peut être conservée pendant une durée comprise entre 6 et 24 mois à une température comprise entre -30°C et -10°C.

Dans un second mode de réalisation, la composition comprenant de la rapamycine, du diméthylsulfoxide et de l'eau telle que décrite plus haut est utilisée dans une administration *in vivo* chez le donneur avant le prélèvement du greffon hépatique.

De préférence, l'administration *in vivo* est une administration par voie intraveineuse.

La présente invention concerne également une composition comprenant de la rapamycine, du diméthylsulfoxide et de l'eau pour utilisation dans le traitement de la stéatose d'un greffon hépatique, susceptible d'être obtenue par un procédé de préparation comprenant les étapes suivantes :
1) Mise en solution de rapamycine avec du diméthylsulfoxide à une température supérieure à 18°C;
2) Ajout d'eau à une température supérieure à 18°C, à la solution de rapamycine et de diméthylsulfoxide obtenue à l'étape 1;
3) Stérilisation de la solution obtenue à l'étape 2, de préférence par filtration.

Le procédé de préparation, par lequel la composition selon l'invention est susceptible d'être obtenue ou obtenue par ledit procédé de préparation, peut comprendre une étape finale dans lequel la solution obtenue à l'étape 2 est traitée avec un gaz inerte tel que de l'azote, l'argon, le dioxyde de carbone, le xénon ou le krypton. Cette étape finale a pour but de limiter le risque de dégradation oxydative de la rapamycine.

De préférence, à l'étape 1 et l'étape 2, la température est supérieure à 18°C et inférieure à 50°C, de préférence comprise entre 19°C et 30°C, plus préférentiellement comprise entre 20°C et 25°C, et en particulier, la température est de 20°C.

De préférence, entre l'étape 2 et l'étape 3, une température supérieure à 18°C et inférieure à 50°C, de préférence comprise entre 19°C et 30°C, plus préférentiellement comprise entre 20°C et 25°C, et en particulier, la température est de 20°C est maintenue afin de tenir compte de l'exothermicité du mélange et des dilations/contractions de volumes liées au mélange lui-même des deux solvants, le DMSO et l'eau, et aux variations de température.

De préférence, le volume de la solution obtenue à l'étape 2 peut être complétée avec du diméthysulfoxide à une température supérieure à 18°C et inférieure à 50°C, de préférence comprise entre 19°C et 30°C, plus préférentiellement comprise entre 20°C et 25°C, et en particulier, la température est de 20°C. En particulier, la quantité de diméthylsulfoxide ajoutée permet d'obtenir une composition comprenant entre 70% et 99% de diméthylsulfoxide, exprimé en pourcentage volumique.

De préférence, la rapamycine est mise en solution dans l'étape 1 à raison de 5 mg/mL.

De préférence, l'étape 3 de stérilisation peut être réalisée par filtration.

De préférence les filtres utilisés sont compatibles avec les solvants utilisés, sans compromettre la teneur en rapamycine. La taille des pores des filtres est comprise de préférence entre 0,1µm et 0,22µm, encore plus préférentiellement de 0,2µm.

De préférence, la filtration est réalisée avec un filtre en nylon ou en polytétrafluoroéthylène. La présente invention porte également sur un procédé d'administration d'une composition telle que décrite ci-dessus, comportant les étapes suivantes :
1) Installation d'un greffon hépatique sur une machine de perfusion en condition hypothermique, puis en condition normothermique;
2) Injection dans le circuit de perfusion de la composition comprenant de la rapamycine, du diméthylsulfoxide et de l'eau.

On entend par « condition hypothermique », une température comprise entre 4°C et 8°C.

De préférence, à l'étape 1, la condition hypothermique est mise en oeuvre à une température comprise entre 4°C et 8°C pendant 1 heure à 2 heures. De préférence, à l'étape 1, la condition normothermique est mise en oeuvre à une température comprise entre 36°C et 38°C pendant 12 heures à 120 heures.

De préférence, entre la condition hypothermique et la condition normothermique de l'étape 1, une phase intermédiaire de réchauffement en condition sub-normothermique, à une température comprise entre 18°C et 22°C, de préférence à une température de 21°C est mise en oeuvre pendant 20 minutes à 1 heure, de préférence pendant 30 minutes.

De préférence, à l'étape 2, l'injection dans le circuit de perfusion de la composition de rapamycine, de diméthylsulfoxide et d'eau est réalisée 2 heures après le début de la condition normothermique.

La présente invention porte également sur l'utilisation d'une composition comprenant de la rapamycine, du diméthylsulfoxide et de l'eau pour traiter la stéatose d'un greffon hépatique.

L'utilisation selon la présente invention comprend avantageusement une composition comprenant de la rapamycine, du diméthylsulfoxide et de l'eau administrée ex *vivo* au greffon hépatique et avantageusement la concentration en rapamycine dans la composition est inférieure ou égale à 1 mg/mL, de préférence entre 0,4 mg/mL et 1 mg/mL. Avantageusement, la concentration circulante en rapamycine est comprise entre 120 ng/mL et 360 ng/mL, de préférence de 180 ng/mL.

Avantageusement, la composition comprend entre 70% et 99% de diméthylsulfoxide et entre 1% et 30% d'eau, de préférence 85% de diméthylsulfoxide et 15% d'eau, exprimé en pourcentage volumique.

Tous les aspects généraux et particuliers décrits plus haut pour la composition comprenant de la rapamycine, du diméthylsulfoxide et de l'eau s'appliquent également pour son utilisation.

D'autres caractéristiques et avantages de l'invention apparaîtront dans les exemples qui suivent, donnés à titre illustratif, avec référence à :

### EXEMPLES

### Exemple 1: Développement d'une solution de rapamycine à 0,5 mg/mL pour injection ex vivo d'un greffon hépatique

### 1. Matériels et Méthodes

**Tableau 1 : Composition qualitative et quantitative de la composition de l'exemple 1.**

| **Composition** | **Quantité** | **Fonction** | **Standards de référence** | **Fabricant/Fournisseur** |
|---|---|---|---|---|
| **Rapamycine** | 500mg | Principe actif | Certificat fabricant (AMRI) | AMRI/INRESA |
| **Diméthylsulfoxide** | 850mL | Agent de solubilisation | Monographie 0763 (PE 11 ème édition) | 1. Gaylord Pharmaceutical / INRESA |
| | | | | 2. Merck / Sigma-Aldrich Chimie |
| **Eau ultra-pure** | Qsp 1000mL | - | - | Millipore Merck |

**Tableau 2 : Conditionnement primaire de la composition de l'exemple 1.**

| **Conditionnement primaire** | **Type** | **Fabricant/Fournisseur** |
|---|---|---|
| **Conditionnement** | Flacon en verre ambré, type I | Schott/ADELPHI |
| **Dispositif de fermeture** | Bouchon en chlorobutyl 4432/50 recouvert d'un film d'élastomère Flurotec^{®} | West pharmaceutical/ADELPHI |

Ce type de conditionnement primaire de la composition a été choisi pour diminuer le risque d'interaction contenant/contenu et de protéger la rapamycine de la lumière.

### 2. Développement de la formulation

Les prérequis galéniques de la solution de rapamycine à développer reposent sur :
- La solubilisation et la stabilisation de la rapamycine afin d'obtenir une concentration cible adéquate après dilution pour i) pouvoir manipuler le produit et ii) limiter la quantité de solvant à injecter.
- La stérilité de la solution et l'absence de recristallisation visible dans un milieu salin.

D'un point de vue biologique, il est attendu une absence d'impact délétère sur la viabilité cellulaire des tissus hépatiques (tranches de foies permettant de garder la structure hépatique) et des hépatocytes issus de foie stéatosique (biocompatibilité), une absence de captation rapide par les hématies (biodisponibilité) et une efficacité de la stimulation du dégraissage sur tissus hépatiques puis sur des foies explantés.

### 2.1 Solubilité et choix du solvant

Plusieurs publications font état de la formulation de rapamycine à partir de liposomes (Rouf MA et al., 2009, Ghanbarzadeh S et al., 2014), de cyclodextrines (Dou Y et al., 2016, Rouf MA et al., 2007), de micelles (Rapamycin encapsulated in dual-responsive micelles for cancer therapy, 2023), de DMSO (commercialisation de la rapamycine solubilisée dans du DMSO jusqu'à 2,5 mg/mL, grade non pharmaceutique) ou par la méthode des cosolvants (Simamora P et al., 2001).

Cependant les formes solubilisées issues de systèmes supramoléculaires et spécialement les liposomes et micelles ont été exclues, de par leur complexité et leur capacité à modifier la distribution d'un principe actif. Le but de la présente invention est de rendre la rapamycine immédiatement disponible pour le greffon hépatique à traiter sur circuit de perfusion.

Le DMSO est un excipient pharmaceutique pour lequel la rapamycine présente une haute solubilité, supérieure à 250 mg/mL (Simamora P et al., 2001).

Le DMSO est utilisé en administration intra-vésicale sous forme de solution aqueuse 50/50 DMSO/H₂O (RIMSO-50^{®}, solution pour instillation intravésicale indiquée pour le traitement des cystites interstitielles) sous une autorisation d'accès compassionnel et est approuvé par la Food and Drug Administration (FDA).

### 2.1.1 Etude de solubilité

Une étude de solubilité a été réalisée sur des mélanges DMSO/H₂O, de 40% à 100% (v/v) en DMSO et des concentrations en rapamycine comprises entre 0,4 et 4 mg/mL.

L'évaluation de la solubilisation de rapamycine en fonction de différentes concentrations de DMSO est présentée au Tableau 3 ci-dessous :

**Tableau 3 : Evaluation de la solubilisation de rapamycine en fonction de différentes concentrations de DMSO.**

| **DMSO/H₂O (% volumique)** | **[Rapamycine] (mg/mL)** | **Solubilité à température ambiante** |
|---|---|---|
| 40 | 0,4 | Non (aspect laiteux avec cristaux |
| | 1 | Non (aspect laiteux avec cristaux |
| | 4 | Non (aspect laiteux avec cristaux |
| 60 | 0,4 | Oui |
| | 1 | Oui |
| | 4 | Non (aspect laiteux avec cristaux |
| 80 | 0,4 | Oui |
| | 1 | Oui |
| | 4 | Oui |
| 100 | 0,4 | Oui |
| | 1 | Oui |
| | 4 | Oui |

Les résultats montrent que la rapamycine est soluble pour l'intervalle de concentration étudiée 0,4 et 4 mg/mL pour des concentrations en DMSO égales ou supérieures à 80% ainsi que pour une concentration de 60% en DMSO pour des concentrations en rapamycine égales ou inférieures à 1 mg/mL (Tableau 3).

A la suite de ces résultats, une nouvelle étude a été lancée sur un intervalle plus restreint en DMSO, avec 3 niveaux de concentrations (70% ; 85% ; 100%) et les 3 mêmes niveaux de rapamycine (0,4 ; 1 ; 4 mg/mL) (Tableau 4).

**Tableau 4 : Evaluation de la solubilisation de rapamycine dans des concentrations de DMSO à 70%, 85% et 100%.**

| **DMSO/H₂O (% volumique)** | **[Rapamycine] (mg/mL)** | **Solubilité à température ambiante** |
|---|---|---|
| 100 | 4 | Oui |
| | 1 | Oui |
| | 0,4 | Oui |
| 85 | 4 | Oui |
| | 1 | Oui |
| | 0,4 | Oui |
| 70 | 4 | Oui |
| | 1 | Oui |
| | 0,4 | Oui |

Les résultats montrent que la rapamycine jusqu'à 4mg/mL est soluble à des concentrations de DMSO de 70% à 100%, exprimé en pourcentage volumique.

### 2.1.2 Evaluation du risque de précipitation

Une évaluation du risque de précipitation des formulations (70% ; 85% ; 100% en DMSO) dans le sang a été réalisée dans des conditions défavorables, couramment dénommées en anglais « worst case ».

On entend par « condition « worst case » », une condition ou un ensemble de conditions défavorables, en comparaison à des conditions idéales, comportant le plus grand risque de défaillance du produit ou du procédé. Cela englobe les circonstances et les limites des procédés (supérieures et inférieures), dans les limites des procédures opératoires.

Les conditions « worst case » pour l'évaluation du risque de précipitation ont été définies comme suit : 1) milieu faiblement accepteur: absence de solvants, de cellules (hématies), de protéines, de lipides ou lipoprotéines pouvant solubiliser la rapamycine ou faire une suspension colloïdale. Ainsi un tampon phosphate salin à pH 7,4°C et à 37°C est jugé faiblement accepteur. 2) Concentration en soluté importante : une concentration en rapamycine supérieure à ce qui est envisagé *in vivo* après dilution présente un risque de précipitation. Les concentrations après dilution de 4 à 40 µg/mL sont bien supérieures à celles envisagées en clinique après dilution dans le circuit de perfusion et sont donc jugées à risque de précipitation.

Pour évaluer ce paramètre, une dilution au 100^{ième} des différentes solutions de rapamycine précédemment présentées a été effectuée dans un tampon phosphate salin maintenu à 37°C, dépourvu de systèmes membranaires de type bicouche lipidique (la rapamycine se distribuant au niveau des éléments cellulaires dans le sang).

L'évaluation du risque de précipitation des solutions dans le sang via dilution au 100^{ième} des différentes solutions dans une solution de tampon phosphate salin à 37°C est présentée au tableau 5 ci-dessous :

**Tableau 5 : Evaluation du risque de précipitation des solutions dans le sang via dilution au 100ème des différentes solutions dans une solution de tampon phosphate salin à 37°C.**

| **DMSO/H₂O (% volumique)** | **[Rapamycine] avant dilution (mg/mL)** | **[Rapamycine] après dilution au 100^{e} (µg/mL)** | **Aspect visuel à température ambiante** |
|---|---|---|---|
| 100 | 4 | 40 | Trouble* |
| | 1 | 10 | Non trouble** |
| | 0,4 | 4 | Non trouble |
| 85 | 4 | 40 | Trouble |
| | 1 | 10 | Non trouble |
| | 0,4 | 4 | Non trouble |
| 70 | 4 | 40 | Trouble |
| | 1 | 10 | Non trouble |
| | 0,4 | 4 | Non trouble |

| | | | |
|---|---|---|---|
| * Recristallisation ** Absence de cristaux (aspect visuel) | | | |

Lors de la dilution dans la solution de tampon phosphate salin, toutes les solutions à 4 mg/mL de rapamycine, quelle que soit la proportion de DMSO, ont induit un trouble du tampon phosphate salin. Il semblerait donc que la rapamycine soit, dans cette concentration, en suspension dans le tampon phosphate salin.

Les solutions égales ou inférieures à 1 mg/mL en rapamycine, quelle que soit la proportion en DMSO, n'ont quant à elles présenté aucun impact sur l'aspect du tampon. D'après l'ensemble des analyses effectuées sur les solutions de rapamycine à base de DMSO, il est ainsi préféré :
- Une concentration minimale de 70% (v/v) en DMSO pour assurer sa solubilisation (Tableau 4).
- Une concentration inférieure ou égale à 1 mg/mL de rapamycine pour limiter le risque de recristallisation après dilution dans le milieu de perfusion (Tableau 5).

### 3. Développement du procédé de fabrication

### 3.1 Etapes du procédé de fabrication réalisés en laboratoire

Les étapes nécessaires à la fabrication de la solution de rapamycine 0,5 mg/mL sont indiquées dans le Tableau 6 ci-dessous :

**Tableau 6 : Procédé de fabrication réalisé en laboratoire.**

| **Etapes** | | **Description** | | **Environnement** | **Matériel(s) utilisé(s)** | | **Appareil utilisé** |
|---|---|---|---|---|---|---|---|
| **1** | **Pesée et préparation de la solution mère** | Solubilisation de la rapamycine à 5mg/mL : | | Salle CMR | | | Balance |
| | | - | peser 50mg de rapamycine | | - | bécher en verre 15 mL | |
| | | - | les disposer dans une fiole de 10mL | | - | cupule | |
| | | - | rincer la cupule avec du DMSO | | - | fiole verre ambrée 10mL | |
| | | | | | - | bouchon fiole | |
| | | - | réaliser le QSP en DMSO | | - | spatule | |
| | | - | homogénéiser par inversion | | | | |
| **2** | **Préparation de la solution finale** | - | ajouter 5mL de la solution mère préalablement préparée dans une fiole de 50mL | Sous hotte | - | béchers en verre 15mL | Non applicable |
| | | - | ajouter 7,5mL d'eau ultra pure | | - | bécher en verre 50mL | |
| | | - | ajouter du DMSO sans atteindre le trait de jauge | | - | seringue combitips advanced^{®} 5-10mL | |
| | | - | refermer et attendre le retour à température ambiante (environ 30 minutes) | | - | fiole verre ambrée 50mL | |
| | | | | | - | bouchon fiole | |
| | | - | réaliser le QSP DMSO | | | | |
| | | - | homogénéiser | | | | |
| **3** | **Filtration** | - | verser la solution dans un bécher | Sous hotte | - | béchers en verre 50mL | Filtration manuelle |
| | | - | prélever à la seringue la solution | | - | seringue plastique 60mL | |
| | | - | filtrer sur nylon ou PTFE 0,2µm dans un bécher | | - | filtre nylon ou PTFE de 0,2µm | |
| **4** | **Remplissage** | - | répartir 2,5mL de la solution filtrée dans le conditionnement primaire | Sous hotte | - | seringue 2,5mL | Non applicable |
| | | | | | - | flacons en verre ambré de type I - 4mL (Schott) | |
| **5** | **Inertage** | - | inerter à l'azote | Sous hotte | - | temps : entre 5 - 10 secondes | Non applicable |
| | | | | | - | pression (Pression réglée sur un manomètre et un ajusteur de débit manuel, en l'absence d'indicateur numérique | |
| **6** | **Scellage** | | | Sous hotte | - | bouchon coaté de 13mm chlorobutyl 4432/50 FluroTec^{®} (Westar) | Système de scellage manuel |
| | | - | boucher les flacons | | | | |
| | | - | sceller | | | | |
| | | | | | - | capsule aluminium | |
| **7** | **Stockage** | - | déposer les échantillons dans un congélateur -20°C | Sous hotte | | NA | Congélateur |

### 3.2 Stérilité de la formulation

La présente invention a pour but de fournir une solution unidose pour une injection lors de la perfusion sur machine du greffon hépatique. La stérilité de la solution doit être conforme aux exigences de la Pharmacopée Européenne.

Afin de respecter les exigences de la Pharmacopée Européenne en matière de stérilité et de réduire les risques de dégradation de la rapamycine par la chaleur ou l'irradiation (caractère thermosensible), la filtration stérilisante sur membrane est la méthode choisie pour réduire la contamination par des particules viables ou non. Le procédé de fabrication inclut donc une étape de filtration stérilisante.

### 3.3 Choix du filtre stérilisant

Les filtres en polytétrafluoroéthylène (PTFE), polypoprylène (PP) et en nylon sont reconnus pour être compatibles avec le DMSO. La filtration stérilisante des solutions a été réalisée avec des filtres stériles de diamètre 25 mm en PTFE et en nylon (taille des pores : 0,2 µm) et filtre Sartoscale^{®} avec membrane en nylon (Sartolon Sartoscale Disposable, taille des pores 0.45 + 0.2 µm).

### 3.3.1 Filtre en PTFE

Les solutions testées ont fait l'objet d'une étude de filtration sur filtre PTFE 0,22 µm (filtre seringue, diamètre 25 mm) après une semaine de stockage à 4°C. La teneur en rapamycine a été évaluée via leurs aires sur les chromatogrammes avant et après filtration lors de la même séquence d'analyse. Il est observé une absence d'impact de la teneur en rapamycine après filtration (Tableau 7). Les solutions sont filtrables sur des filtres PTFE 0,22 µm.

**Tableau 7 : Evaluation de l'impact de la filtration sur filtre PTFE 0,22 µm sur la teneur en rapamycine.**

| **DMSO (% volumique)** | **[rapamycine] (mg/mL)** | **Ratio des aires de rapamycine avant/après filtration (%)** |
|---|---|---|
| 100 | 4 | 102% |
| | 1 | 99% |
| | 0,4 | 99% |
| 85 | 4 | 100% |
| | 1 | 101% |
| | 0,4 | 100% |
| 70 | 4 | 99% |
| | 1 | 100% |
| | 0,4 | 99% |

### 3.3.2 Filtre en nylon

### - Filtre stérilisant Nalgene^{®}

Le filtre sélectionné pour la réalisation des essais indicateurs de pré-stabilité et de stabilité est le filtre stérilisant 0,2 µm en nylon Nalgene^{®} du fournisseur Thermo Scientific.

Les teneurs en rapamycine après filtration sont conformes aux spécifications.

### - Filtre stérilisant Sartoscale^{®}

Le filtre sélectionné pour tester l'intégrité du filtre est le filtre stérilisant 0,2 µm Sartoscale^{®} (membrane en nylon, corps en polypropylène) du fournisseur Sartorius.

L'intégrité après filtration stérilisante a été réalisée par la méthode du test de point de bulle à l'eau. Le test est conforme aux spécifications dans les conditions de réalisation du laboratoire.

### 3.4 Détermination des conditions de conservation et de stockage

### - Etude de pré-stabilité

Les essais de pré-stabilité des formules ont été mis en place pour permettre de sélectionner la formule finale. Ils ont été réalisés dans les conditions suivantes :
- Trois niveaux de concentration de DMSO : 70% ; 85% ; 100% (v/v).
- Deux niveaux de concentration de rapamycine : 133 µg/mL et 500 µg/mL.
- Echéances : T0, T1, T2, T3, T6, T9 et T12 mois.
- Températures : + 4°C et - 20°C.

La concentration à 133µg/mL est la concentration la plus basse choisie permettant de détecter les substances apparentées.

Les 6 formules testées sont représentées dans le Tableau 8 ci-dessous :

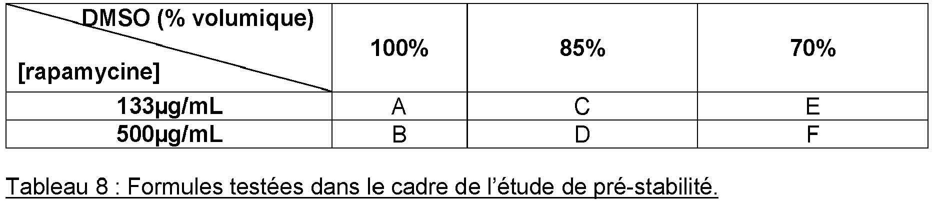

- Dès T0, quelles que soient les conditions de stockage (+ 4°C et - 20°C), on observe l'apparition des impuretés supérieurs au seuil de report pour les formules A et B.
- Dès T0, quelles que soient les conditions de stockage (+ 4°C et -20°C), les dosages de rapamycine sont non-conformes (<95%) pour les formules E et F.
- Jusqu'à T6 mois, la formule C est stable au congélateur (-20°C),
- Jusqu'à T9 mois, les formules E, F et D sont stables au congélateur (-20°C). Cependant, dès T0 mois, les formules E et F présentent une teneur en rapamycine inférieure à 95%.
- A T9 mois, pour le stockage au congélateur (-20°C), une impureté apparait au-dessus du seuil de report pour la formule C ; cette impureté n'apparaît pas pour la formule D.
- A T9 mois, la formule D stockée à -20°C reste stable. En revanche, une impureté apparaît au-dessus du seuil de report pour la formule D stockée à 4°C.

Il apparait que la formule D (concentration en rapamycine de 500 µg/mL ; 85% DMSO : 15% eau) stockée à -20°C soit la plus stable. De plus, le mélange reste sous forme liquide à -20°C pour cette proportion DMSO:eau, présentant un intérêt pour une utilisation rapide au bloc opératoire. Le mélange exothermique DMSO:eau n'a pas impacté la stabilité de la rapamycine.

### - Etude de stabilité en conditions normales d'utilisation

Afin de reproduire les conditions des essais de réalisation de perfusion des foies stéatosiques au bloc opératoire et de déterminer les conditions de conservation de la formule à la sortie du stockage à -20°C, des études de stabilité en conditions normales d'utilisation ont été réalisées sur des flacons fermés et ouverts à 6 échéances (T0, T2h, T5h, T7h, T17h et T24h) à 20°C+/- 5°C.

Les études de pré-stabilité et de stabilité en conditions normales d'utilisation ont ainsi permis de déterminer les conditions de conservation. La composition est conservée et stockée au congélateur de préférence à -20°C. A cette température, le produit reste liquide. A la sortie du congélateur, et dès son retour à température ambiante, il est préconisé d'utiliser les flacons immédiatement (entre 2h et 7h).

### 3.5 Paramètres critiques du procédé de fabrication

Les paramètres critiques du procédé de fabrication ont été identifiés et sont indiqués dans le Tableau 9 ci-dessous :

**Tableau 9 : Paramètres critiques du procédé de fabrication.**

| **Paramètre critique** | | **Plage de liberté** | **Objectif** |
|---|---|---|---|
| **Environnement** | Température de fabrication | <50°C | Stabilité rapamycine |
| | | >18°C | Solidification du DMSO à 18°C |
| **Environnement** | Lumière environnement de fabrication | Filtres à identifier | Garantir la stabilité de la rapamycine |
| **Fabrication** | Matière des équipements utilisés Polymères | PTFE - jusqu'à 189°C Polyéthylène Polypropylène Ethylène propylène gomme - jusqu'à 48°C | Limiter la solubilisation du DMSO avec de nombreux composés |
| **Fabrication** | Matière des équipements utilisés métaux | 304-316 acide inoxydable | Niveau de corrosion très faible du DMSO |
| **Fabrication** | Thermostat ou système d'ajout lent de l'eau au DMSO | ΔH | Limiter la réaction exothermique |
| **Fabrication** | Filtre | Nylon ou PTFE | Stériliser le produit et éliminer d'éventuels contaminants particulaires |
| **Stockage** | Stockage du produit fini | -20°C | Limiter la dégradation de la rapamycine |
| **Stockage** | Température de stockage du DMSO | >18°C | Solidification du DMSO à 18°C |

### Exemple 2 : Evaluation de l'effet « dégraissant » de la rapamycine

On entend par effet « dégraissant » notamment une diminution du taux de lipides et/ou de triglycérides au niveau hépatique.

### 1. Résultats in vitro

La rapamycine a été évaluée sur des cultures d'hépatocytes et sur tissus hépatiques (tranches de foies permettant de garder la structure hépatique). Pour une concentration de rapamycine à 200nM, il était observé une diminution de 19% du taux de triglycérides intracellulaires par rapport au contrôle dans les hépatocytes et de 38% dans les tissus hépatiques. Ces résultats répétés plusieurs fois ont permis de confirmer la preuve de concept de l'effet de la rapamycine sur l'effet « dégraissant ».

### 2. Résultats sur circuit de perfusion sans organe

Une machine de perfusion hépatique a été utilisée avec du perfusat constitué de sang total humain maintenu à 37°C. La rapamycine a été injectée directement dans le circuit de perfusion. Des prélèvements étaient réalisés à intervalles réguliers pour doser la molécule. L'expérience a été réalisée 4 fois, l'objectif étant de valider la concentration cible théorique de 180 ng/mL définie à partir des études précédentes d'efficacité et de toxicité.

Les résultats ont montré l'obtention d'une concentration moyenne de rapamycine de 150 +/- 30 ng/mL ce qui valide la première étape.

### 3. Résultats sur circuit de perfusion avec foie de cochon

Le même protocole de perfusion a été utilisé mais avec un foie animal. Celui-ci a été prélèvé chez un cochon en arrêt cardiaque puis préservé dans de la glace. Il a ensuite été mis sur machine de perfusion, initialement en hypothermie (4°C) pendant 2 heures puis à 37°C pendant 12 heures avec une phase de réchauffement intermédiaire (sub normothermie, 21°C). La rapamycine était injectée dans le circuit 2 heures après le début de la phase de normothermie. Neuf expériences ont été réalisées. L'objectif était d'avoir une étude pharmacocinétique de la rapamycine.

Les dosages de la rapamycine dans le sang total et le plasma montrent un pic de concentration 5 minutes après l'injection de rapamycine dans le circuit puis une décroissance progressive au cours du temps. La rapamycine a été également dosée dans la bile et elle est retrouvée dès 1 heure après l'injection dans le sang pour augmenter au fur et à mesure de la perfusion.

Ces résultats montrent donc une captation de la rapamycine par le foie et son métabolisme avec excrétion dans la bile.

### 4. Résultats sur circuit de perfusion avec foie humains

De la même manière, l'expérience a été réalisée avec deux foies humains jugés non transplantables et utilisés à visée scientifique après autorisation de l'Agence de Biomédecine. Ces deux foies présentaient une stéatose significative (supérieure à 50%). L'évaluation de la viabilité de ces deux organes ont été faites selon des critères validés en pratique clinique. L'objectif était d'évaluer l'effet de la rapamycine sur la stéatose. Pour ce faire, les triglycérides intra cellulaires ont été dosés.

Concernant la pharmacocinétique, celle-ci était identique à celle observée lors de la perfusion des foies de cochon.

Pour la stéatose, le taux de triglycérides intra cellulaires a diminué de 39% 9 heures après l'injection de rapamycine pour le foie N°1 et de 31% pour le foie N°2.

### Exemple 3 : Evaluation de la toxicité et de l'efficacité de la solution de rapamycine

Les essais de solubilisation et de stabilité tels que détaillés en exemple 1 ont permis d'identifier deux formules pour les essais in vitro :
- DMSO 85%:eau15% jusqu'à 1mg/mL en rapamycine (ci-après « Rapa-85% »); et
- DMSO 100% jusqu'à 1mg/mL en rapamycine (ci-après « Rapa-100% »).

Les formules ont été comparées à concentration équivalente en rapamycine lors de tests de toxicité et efficacité cellulaire sur deux modèles :
▪ Modèle *in vitro* (2D) : hépatocytes humains en culture primaire
   - Evaluation de l'effet de la rapamycine dans une solution Rapa-100% ;
   - Comparaison de l'effet dégraissant de la rapamycine dans 2 formules : Rapa-100% et Rapa-85% pour confirmer l'efficacité de l'effet dégraissant de la rapamycine sur la stéatose développée in vitro.
▪ Modèle ex *vivo* (3D) : tissus hépatiques ou tranches de foie humaines coupées à la précision (human Precision-Cut Liver slices (hPCLS)) en culture primaire.

La toxicité des formules a été évaluée par la viabilité cellulaire des hépatocytes et leur efficacité par le suivi de la concentration intracellulaire en triglycérides (TG). L'objectif de ces essais est de sélectionner la formule adaptée et la dose de rapamycine.

Les résultats in vitro sont indiqués dans le Tableau 10 ci-dessous :

**Tableau 10 : Résultats sur les modèles in vitro 20.**

| **Essais réalisés** | **Efficacité** | | **Toxicité** | **Concentration en rapamycine testée** |
|---|---|---|---|---|
| **Modèle *in vitro :* Evaluation de l'effet de la rapamycine dans une solution Rapa-100%** | - | Diminution des gouttelettes lipidiques de 30% dans les hépatocytes traités par la rapamycine par rapport aux hépatocytes stéatosiques. | Aucun effet global sur la viabilité des hépatocytes humaines n'a été constaté. | 200nM |
| | - | Diminution de la teneur en triglycérides (TG) intracellulaires de 24% (p<0,05) dans les hépatocytes stéatosiques traités par rapport aux hépatocytes stéatosiques. | | |
| **Modèle *in vitro :* Comparaison de l'effet dégraissant de la rapamycine dans 2 formules : Rapa-100% et Rapa-85%, sur des hépatocytes issus de foie stéatosique** | Pour des [Rapa-85%] à 200 nM, 500 nM ou 1000 nM : | | Aucun effet global sur la viabilité des hépatocytes humaines n'a été constaté pour la formule [Rapa-85%] et [Rapa-100%] à différentes concentrations. | |
| | - | Diminution des gouttelettes lipidiques intracellulaires de 24%, 25% ou 28% respectivement (p<0,05) par rapport aux hépatocytes stéatosiques traités. | | |
| | - Diminution des TG intracellulaires de 19%, 20% ou 10% respectivement (p<0,05) par rapport aux hépatocytes stéatosiques traités. | | | 20nM |
| | | | | 200 nM |
| | | | | 500 nM |
| | | | | 1000 nM |
| | Pour des [Rapa-100%] à 200 nM : diminution des gouttelettes lipidiques et des TG intracellulaires de 19% et de 20% par rapport aux cellules traitées avec le véhicule. | | | |

Les résultats montrent que l'ajout de rapamycine n'a pas d'impact sur la viabilité des hépatocytes.

De plus, les résultats montrent que l'effet dégraissant de la rapamycine sur des hépatocytes issus de foie stéatosique à plusieurs concentrations n'altèrent pas la viabilité cellulaire.

Les résultats *in vitro* montrent que les solutions [Rapa-100%] ou [Rapa-85%] diminuent significativement le niveau de gouttelettes lipidiques et de TG intracellulaires d'hépatocytes stéatosiques, à différentes concentrations en rapamycine. Ils ont également permis de confirmer l'efficacité de [Rapa-85%] et [Rapa-100%] pour dégraisser les hépatocytes stéatosiques sans altérer la viabilité cellulaire.

Les résultats ex *vivo* sont indiqués dans le Tableau 11 ci-dessous :

**Tableau 11 : Résultats sur les modèles ex vivo 3D.**

| **Essais réalisés** | **Efficacité** | | **Toxicité** | **Concentration en rapamycine testée** |
|---|---|---|---|---|
| **Modèle ex *vivo* (3D) *: tissus* hépatiques (Précision eut liver slices (PCLS))** | - | Diminution significative de la teneur en TG dans les PCLS de 39 % et 38 % par rapport au véhicule (p<0,05), respectivement pour [Rapa-100] et [Rapa-85%] à une concentration de 200 nM. | Aucun effet global sur la viabilité des *slices* hépatiques n'a été observé pour les 2 formules quelle que soit la concentration utilisée. | |
| **- [Rapa-100%] et** | | | | |
| **- [Rapa-85%]** | | | | 20 nM |
| | | | | 200 nM |
| | | | | 500 nM |
| | - | Diminution significative des TG intracellulaires de 40% des PCLS traités avec [Rapa-100%] à 500 nM contrairement aux PCLS traités avec [Rapa-85%] à la même dose (p<0,05). | | 1000 nM |

Les résultats ex *vivo* montrent que les solutions [Rapa-100%] ou [Rapa-85%] diminuent significativement le niveau de TG intracellulaire à la concentration cible dans le sang de 200 nM. Ils ont également permis de confirmer l'efficacité de [Rapa-85%] et [Rapa-100%] pour dégraisser les PCLS stéatosiques sans altérer la viabilité cellulaire.

La forme liquide à -20°C de la solution [Rapa-85%] (intérêt pour une utilisation rapide au bloc opératoire) et les résultats de pré-stabilité décrits précédemment ont permis de sélectionner cette formule.

### Exemple 4 : Compositions comprenant de la rapamycine, du diméthylsulfoxide et de l'eau selon la présente invention

Le tableau 12 ci-dessous représente différentes formulations de la composition selon la présente invention :

**Tableau 12 : Différentes formulations de la composition selon la présente invention.**

| | | | | | | | | | |
|---|---|---|---|---|---|---|---|---|---|
| Concentration rapamycine (µg/mL) | 400 | 400 | 400 | 500 | 500 | 500 | 4000 | 4000 | 4000 |
| Pourcentage volumique DMSO (%) | 70 | 85 | 99 | 70 | 85 | 99 | 70 | 85 | 99 |
| Pourcentage massique rapamycine (%) | 0,04 | 0,04 | 0,04 | 0,05 | 0,04 | 0,05 | 0,37 | 0,37 | 0,36 |
| Pourcentage massique DMSO (%) | 72,60 | 86,45 | 98,87 | 72,60 | 86,44 | 98,87 | 72,36 | 86,16 | 98,56 |
| Pourcentage massique Eau (%) | 27,36 | 13,51 | 1,09 | 27,35 | 13,52 | 1,08 | 27,27 | 13,47 | 1,08 |
| Ratio DMSO/Eau (m/m) | 2,65 | 6,40 | 91,25 | 2,65 | 6,40 | 91,25 | 2,65 | 6,40 | 91,25 |

### REFERENCES

Li J, Kim SG, Blenis J. Rapamycin: one drug, many effects. Cell Metab. 4 mars 2014; 19(3):373-9.
Muller X, Rossignol G, Mohkam K, Mabrut JY. Stratégies de conservation des greffons en transplantation hépatique - progrès et perspectives en France. J Chir Viscérale. 1 oct 2022;159(5):412-22.
Boteon YL, Wallace L, Boteon APCS, Mirza DF, Mergental H, Bhogal RH, et al. An effective protocol for pharmacological defatting of primary human hépatocytes which is nontoxic to cholangiocytes or intrahepatic endothélial cells. Vinciguerra M, éditeur. PLOS ONE. 25 juill 2018;13(7):e0201419.
Rouf MA, Vural I, Renoir JM, Hincal AA. Development and characterization of liposomal formulations for rapamycin delivery and investigation of their antiproliferative effect on MCF7 cells. J Liposome Res. 2009;19(4):322-31.
Ghanbarzadeh S, Khorrami A, Mohamed Khosroshahi L, Arami S. Fusogenic pH sensitive liposomal formulation for rapamycin: improvement of antiproliferative effect. Pharm Biol. juill 2014;52(7):848-54.
Dou Y, Guo J, Chen Y, Han S, Xu X, Shi Q, et al. Sustained delivery by a cyclodextrin material-based nanocarrier potentiates antiatherosclerotic activity of rapamycin via selectively inhibiting mTORC1 in mice. J Control Release Off J Control Release Soc. 10 août 2016;235:48-62.
Rouf MA, Bilensoy E, Vural I, Hincal A. Abdur Rouf M, Vural I, Bilensoy E, Hincal A, Erol DD. Rapamycin-cyclodextrin complexation: improved solubility and dissolution rate. J Incl Phenom Macrocycl Chem. 1 juin 2011;70(1):167 75. Eur J Pharm Sci - EUR J PHARM SCI. 1 sept 2007;32.
Rapamycin encapsulated in dual-responsive micelles for cancer therapy - ScienceDirect [Internet]. [cité 13 févr 2023]. Disponible sur: https://www.sciencedirect.com/science/article/pii/S0142961212011556
Simamora P, Alvarez JM, Yalkowsky SH. Solubilization of rapamycin. Int J Pharm. 1 févr 2001;213(1-2):25-9

## Revendications

1. Composition comprenant de la rapamycine, du diméthylsulfoxide et de l'eau pour utilisation dans le traitement de la stéatose d'un greffon hépatique.

2. Composition selon la revendication 1, dans laquelle la composition est sous forme de solution injectable.

3. Composition selon la revendication 1 ou 2, dans laquelle la composition est utilisée dans une administration ex *vivo* au greffon hépatique.

4. Composition selon la revendication 3, dans laquelle l'administration ex *vivo* est une perfusion en condition normothermique.

5. Composition selon la revendication 3 ou 4, dans laquelle la concentration en rapamycine dans la composition est inférieure ou égale à 1 mg/mL, de préférence entre 0,4 mg/mL et 1 mg/mL, de préférence de 0,5 mg/mL.

6. Composition selon l'une quelconque des revendications 3 à 5, dans laquelle la concentration circulante en rapamycine est comprise entre 120 ng/mL et 360 ng/mL, de préférence de 180 ng/mL.

7. Composition selon l'une quelconque des revendications 3 à 6, comprenant entre 70% et 99% de diméthylsulfoxide et entre 1% et 30% d'eau, de préférence 85% de diméthylsulfoxide et 15% d'eau, exprimé en pourcentage volumique.

8. Composition selon la revendication 7, dans laquelle le ratio dimethylsulfoxide/eau est compris entre 2,5 et 95, de préférence entre 2,65 et 91,25.

9. Composition selon l'une quelconque des revendications 1 à 8, dans laquelle la stéatose du greffon hépatique est supérieure à 30%.

10. Composition selon l'une quelconque des revendications 1 à 9, dans laquelle la composition est conservée entre -30°C et -10°C, de préférence à -20°C.

11. Composition selon la revendication 1 ou 2, dans laquelle la composition est utilisée dans une administration *in vivo* au greffon hépatique.

12. Composition selon l'une quelconque des revendications 1 à 11, dans laquelle la composition est susceptible d'être obtenue par un procédé de préparation comprenant les étapes suivantes :
1) Mise en solution de rapamycine avec du diméthylsulfoxide à une température supérieure à 18°C;
2) Ajout d'eau à une température supérieure à 18°C, à la solution de rapamycine et de diméthylsulfoxide obtenue à l'étape 1;
3) Stérilisation de la solution obtenue à l'étape 2, de préférence par filtration.

13. Composition selon la revendication 12, dans laquelle la rapamycine est mise en solution dans l'étape 1 à raison de 5 mg/mL.

14. Composition selon la revendication 12 ou 13, dans laquelle la filtration est réalisée avec un filtre en nylon ou en polytétrafluoroéthylène.

15. Procédé d'administration d'une composition telle que définie dans l'une quelconque des revendications 1 à 10 et 12 à 14, comportant les étapes suivantes :
1) Installation d'un greffon hépatique sur une machine de perfusion en condition hypothermique, de préférence entre 4°C et 8°C pendant 1 à 2 heures, puis en condition normothermique, de préférence entre 36°C et 38°C pendant 12 à 120 heures;
2) Injection dans le circuit de perfusion de la composition comprenant de la rapamycine, du diméthylsulfoxide et de l'eau, de préférence 2 heures après le début de la condition normothermique.

16. Utilisation d'une composition comprenant de la rapamycine, du diméthylsulfoxide et de l'eau pour traiter la stéatose d'un greffon hépatique.

17. Utilisation d'une composition selon la revendication 16, dans laquelle la composition est administrée ex *vivo* au greffon hépatique.

18. Utilisation d'une composition selon la revendication 17, dans laquelle la concentration en rapamycine dans la composition est inférieure ou égale à 1 mg/mL, de préférence entre 0,4 mg/mL et 1 mg/mL, encore plus préférentiellement de 0,5 mg/mL.

19. Utilisation d'une composition selon la revendication 17 ou 18, dans laquelle la concentration circulante en rapamycine est comprise entre 120 ng/mL et 360 ng/mL, de préférence de 180 ng/mL.

20. Utilisation d'une composition selon l'une quelconque des revendications 16 à 19, dans laquelle la composition comprend entre 70% et 99% de diméthylsulfoxide et entre 1% et 30% d'eau, de préférence 85% de diméthylsulfoxide et 15% d'eau, exprimé en pourcentage volumique.

21. Utilisation d'une composition selon l'une quelconque des revendications 16 à 20, dans laquelle la stéatose du greffon hépatique est supérieure à 30%.
